# EUROPÄISCHE PATENTANMELDUNG

(11) **EP 2 198 792 A1**
(43) Veröffentlichungstag der Anmeldung: **23.06.2010**
(21) Anmeldenummer: 08405310.7
(22) Anmeldetag: 19.12.2008
(51) Int. Cl.: A61B 17/70, A61B 17/00, A61B 17/60, A61B 17/72

(54) **Implantatsystem zum Stabilisieren von Knochen**

(71) Anmelder: Sepitec Foundation, 9490 Vaduz (LI)
(72) Erfinder: Fiechter, Meinrad, 3110 Münsingen (CH); Magerl, Friedrich, 9011 St. Gallen (CH); Wieling, Ronald, 9453 Eichberg (CH)
(74) Vertreter: Groner, Manfred

(57) **Zusammenfassung**

Das Implantatsystem besitzt wenigsten ein Bauteil (6, 24, 33, 39), das einen länglichen Körper (15, 25, 34, 40) aufweist, der wenigstens einen Abschnitt besitzt, an dem er mit einem Befestigungssystem (2, 3; 42) des Implantatsystems, beispielsweise einer Pedikelschraube oder einer Klammer verbindbar ist. Der längliche Körper (15, 25, 34, 40) ist aus einem röntgentransparenten Kunststoff hergestellt und im genannten wenigstens einen Abschnitt fest mit einem wesentlich härteren Teil (16, 17, 27, 28, 35, 37, 41) verbunden, welcher härtere Teil eine Schnittstelle zum genannten Befestigungselement (2, 42) bildet.

## Beschreibung

Die Erfindung betrifft ein Implantatsystem zum Stabilisieren von Knochen, mit einem Bauteil, das einen länglichen Körper aufweist, der wenigstens einen Abschnitt besitzt, an dem er mit einem an deren Teil, beispielsweise einer Pedikelschraube oder einer Klammer verbindbar ist.

Solche Implantatsysteme werden beispielsweise zum Stabilisieren von Abschnitten der Wirbelsäule verwendet. Diese Systeme umfassen einen als Stab oder Platte ausgebildeten länglichen Körper, der mit wenigstens zwei Pedikelschrauben in Wirbelkörpern verankert wird. Im Stand der Technik sind solche Implantatsysteme in zahlreichen Ausführungen bekannt und beispielsweise in der US 5,474,555 und der EP-A-0 746 255 offenbart. Bei diesen Systemen bilden die genannten Stäbe jeweils Bauteile und die Pedikelschrauben Befestigungselemente. Die Stäbe sind beispielsweise aus Titan, Implantatenstahl oder einem andere biokompatiblen Metall hergestellt. Nachteilig sind bei diesen die mangelnde Röntgentransparenz und die Bildung von Artefakten. Für einige Anwendungen ist zudem das E-Modul zu hoch. Bekannt sind auch Stäbe oder Platten aus Kunststoff, insbesondere PEEK und Kohlenfaser verstärktes PEEK. Diese sind röntgentransparent und erzeugen keine Artefakte. Zudem besitzen sie in der Regel ein E-Modul nahe demjenigen von Knochen. Die Festigkeit sowie die Härte sind jedoch vielfach zu gering. Nachteilig ist auch der Abrieb an den Befestigungsabschnitten, der Teile von Fasern oder der Matrix enthalten kann.

Durch die WO 2006/118866 ist ein Fixationssystem bekannt geworden, das einen Stab aufweist, der aus Metall und Kunststoff hergestellt ist. Der Stab soll den Vorteil besitzen, dass seine Biegefestigkeit und sein Elastizitätsmodul durch entsprechende unterschiedliche Ausbildung der beiden Werkstoffe verändert werden kann. Nachteilig ist aber auch hier die mangelnde Röntgentransparenz.

Bekannt sind auch Implantatsysteme, die einen Marknagel, beispielsweise einen intramodulären Hüftnagel aufweisen. Diese bildet ebenfalls ein längliches Bauteil, das beispielsweise mit einer Schenkelhalsschraube verbunden werden muss. Auch hier ergeben sich die obengenannten Nachteile, insbesondere die mangelnde Röntgentransparenz und die Bildung von Artefakten. Ist der Marknagel ein Stahlnagel, so besteht auch hier der Nachteil, dass sein E-Modul wesentlich grösser ist als der zu stabilisierende Knochen.

Der Erfindung liegt die Aufgabe zu Grunde, ein Implantatsystem der genannten Art zu schaffen, dass die genannten Nachteile vermeidet.

Die Aufgabe ist bei einem gattungsgemässen Implantatsystem dadurch gelöst, dass der längliche Körper aus einem röntgentransparenten Kunststoff hergestellt ist und im genannten wenigstens einen Abschnitt fest mit einem weiteren Teil verbunden ist, welcher weitere Teil eine Schnittstelle zum genannten anderen Teil bildet. Beim erfindungsgemässen Implantatsystem wird somit ein Bauteil verwendet, bei dem lediglich die Abschnitte nicht röntgentransparent sind, an welchen das Bauteil beispielsweise mit einem Befestigungselement, einer Pedikelschraube, einer Schenkelhalsschraube oder einem Fixateur über einen Pin verbunden wird. An diesen Abschnitten bzw. Schnittestellen kann das Bauteil mit vergleichsweise hoher Kraft beispielsweise geklemmt werden. Die Klemmung findet am wesentlichen härteren Teil statt. Ein Abrieb des Kunststoffs kann damit vermieden werden. Da das Bauteil ausserhalb der Befestigungsabschnitte aus Kunststoff hergestellt ist, ist dieser weitgehend röntgentransparent und die Bildung von Artefakten kann wesentlich vermindert werden. Ausserhalb der Befestigungsabschnitte besitzt zudem das Bauteil ein E-Modul, das demjenigen des zu stabilisierenden Knochens entspricht.

Nach einer Weiterbildung der Erfindung ist vorgesehen, dass der härtere Teil an wenigstens einem Ende des länglichen Körpers angeordnet ist. Hierbei ist insbesondere vorgesehen, dass der röntgentransparente Kunststoff ein E-Modul aufweist, das näher beim E-Modul des Knochens ist als der härtere Teil. Eine besonders stabile Befestigung des Befestigungselementes am Bauteil ist dann möglich, wenn der weitere Teil aus Metall, insbesondere Titan, einer Titanlegierung, Implantatenstahl oder Kunststoff hergestellt ist. Dieser härtere Teil kann vergleichsweise kurz ausgebildet sein. Er ist gemäss einer Weiterbildung der Erfindung hülsenförmig ausgebildet. Die Hülse ist fest mit dem aus Kunststoff hergestellten länglichen Körper verbunden. Die Verbindung kann beispielsweise eine Presspassung oder eine Pressung sein. Eine Verbindung ist auch mittels einem Gewinde oder durch eine Schweissverbindung möglich. Ferner kann der Teil aus Kunststoff an den härteren Teil angespritzt sein.

Eine besonders stabile Verbindung ist dann gewährleistet, wenn gemäss einer Weiterbildung der Erfindung der weitere Teil aussenseitig für einen Formschluss mit dem Befestigungselement strukturiert ist. Mit einem solchen Formschluss kann insbesondere eine radiale und/oder axiale relative Bewegung vermieden werden. Die Strukturierung kann insbesondere durch eine Mehrzahl von Vertiefungen oder Erhöhungen realisiert werden. Vorzugsweise weist das Befestigungselement korrespondierende Vertiefungen oder Erhöhungen auf.

Weitere vorteilhafte Merkmale ergeben sich aus den abhängigen Patenansprüchen, der nachfolgenden Beschreibung sowie der Zeichnung.

Ausführungsbeispiele der Erfindung werden nachfolgend anhand der Zeichnung näher erläutert. Es zeigen:
- Figur 1: schematisch eine Ansicht eines erfindungsgemässen Implantatsystems, das einen Abschnitt einer Wirbelsäule stabilisiert,
- Figur 2: ein Schnitt durch einen Stab des Implantatsystems nach Figur 1,
- Figur 3: ein Schnitt durch einen Stab gemäss einer Variante,
- Figur 4: ein Schnitt durch einen Stab gemäss einer weiteren Variante,
- Figuren 5 - 9: Schnitte durch Hülsen gemäss unterschiedlichen Varianten,
- Figur 10: schematisch ein Schnitt durch einen eine Pedikelschraube aufweisenden Anker zum Befestigen eines Stabes an einem Wirbelkörper,
- Figur 11: eine Ansicht des Ankers und ein Abschnitt eines Stabes,
- Figur 12: ein Schnitt durch einen Anker und einen Stab gemäss einer Variante,
- Figur 13: eine Ansicht des Ankers und eines Teilabschnittes des Stabes gemäss Figur 12,
- Figur 14: ein Schnitt durch einen Marknagel,
- Figur 15: ein Schnitt durch einen Marknagel gemäss einer Variante,
- Figur 16: ein Schnitt durch einen intramedulären Hüftnagel,
- Figur 17: ein Längsschnitt durch einen Stab nach einer weiteren Variante,
- Figur 18: ein Längsschnitt durch einen Stab nach einer weiteren Variante und
- Figur 19: ein Querschnitt durch einen Stab nach einer weiteren Variante.

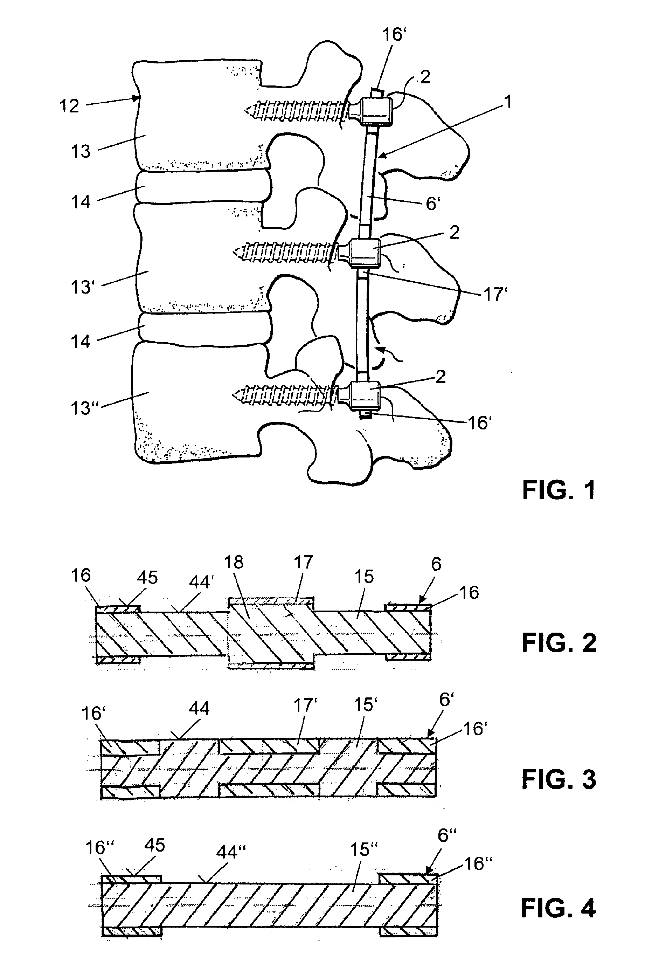

Die Figur 1 zeigt eine Stabilisierungsanordnung 1 bzw. ein Implantatsystem, mit dem drei Wirbel 13, 13' und 13" einer Wirbelsäule 12 stabilisiert werden. Zwischen den Wirbeln 13, 13' und 13" befinden sich die Bandscheiben 14, in denen hier nicht gezeigte Zwischenwirbelelemente eingesetzt sein können. Die Stabilisierungsanordnung 1 soll insbesondere ein Zusammenwachsen der Wirbel 13, 13' und 13" ermöglichen. Die Stabilisierungsanordnung 1 umfasst drei Pedikelschrauben 2, die gleich ausgebildet sein können sowie einen Verbindungsstab 6', der gerade oder gebogen sein kann. In der Regel umfasst die Stabilisierungsanordnung 1 bzw. das Implantatsystem einen weiteren Verbindungsstab 6', der hier verdeckt ist und ebenfalls mit drei Pedikelschrauben 2 verankert ist. Die Verbindungsstäbe 6' sind Bauteile der Stabilisierungsanordnung 1 und verbinden die drei Wirbel 13, 13' und 13" miteinander.

Der in Figur 3 gezeigte Verbindungsstab 6' besteht aus einem länglichen Körper 15', der aus einem vergleichsweise leichten Material, insbesondere aus Kunststoff hergestellt ist. Der Kunststoff ist beispielsweise PEEK oder kohlenfaserverstärktes PEEK, PEK oder ein ähnlicher Werkstoff. Dieser längliche Körper 15' ist fest mit zwei äusseren Hülsen 16' und einer mittleren Hülse 17' verbunden. Diese Hülsen 16' und 17' bestehen aus einem Material, das beispielsweise wesentlich härter ist als das Material des Körpers 15'. Aussenseitig sind die Hülsen 16' und 17' bündig mit einer Aussenseite 44 des Verbindungsstabes 6'. Im Querschnitt ist der Verbindungsstab 6' vorzugsweise kreisrund, es ist aber auch ein anderer Querschnitt, beispielsweise ein ovaler oder polygonaler Querschnitt möglich. Die Hülsen 16' und 17' bestehen insbesondere aus Titan, einer Titanlegierung oder aus einem Implantatenstahl. Sie bilden die Schnittstelle bzw. die Befestigungsabschnitte, an denen der Verbindungsstab 6' mit Klemmvorrichtungen 3 verbunden ist. Die Verbindung ist insbesondere eine Klemmverbindung, vorzugsweise formschlüssige Klemmverbindung. Ausserhalb dieser Abschnitte bildet wie ersichtlich der Körper 15' die Aussenseite. Zwischen den Hülsen 16' und 17' ist der Verbindungsstab 6' somit röntgentransparent. Da der Verbindungsstab 6' an den Hülsen 16' und 17' befestigt ist, ist der Körper 15' beispielsweise durch Klemmkräfte im wesentlichen nicht belastet. Insbesondere können unerwünschte Abriebpartikel, beispielsweise Fasern oder eine Matrix des Körpers 15' nicht erzeugt werden. Solche Abriebpartikel können zu unerwünschten Reaktionen führen.

Der in Figur 2 gezeigte Verbindungsstab 6 unterscheidet sich von demjenigen gemäss der Figur 3 im wesentlichen dadurch, dass der Körper 15 eine Aussenseite 44' aufweist, welche bezüglich wenigstens einer Aussenseite 45 einer Hülse 16 vertieft ist. Zudem ist beim Verbindungsstab 16 etwa mittig eine Verdickung 18 vorgesehen, auf welcher eine Hülse 17 befestigt ist. Der Aussendurchmesser der Hülse 17 ist zudem grösser als der Durchmesser der Hülsen 16.

Schliesslich zeigt die Figur 4 einen Verbindungsstab 16", bei dem auf einem Körper 15" lediglich zwei an den Enden angeordnete Hülsen 16" vorgesehen sind. Dieser Verbindungsstab 6" wird lediglich mit zwei Pedikelschrauben 2 verbunden. Zwischen den Hülsen 16" besteht hier somit ein vergleichsweise grosser Bereich, in welchem der Verbindungsstab 6" röntgentransparent ist. In diesem Bereich entspricht das E-Modul demjenigen des Körpers 15". Denkbar sind hier aber auch Ausführungen, bei welcher mehr als drei Hülsen 16" vorgesehen sind. Beim Verbindungsstab 6" gemäss Figur 4 kann die Aussenseite 44" des Körpers 15" bündig zur Aussenseite 45" der Hülsen 16" sein.

Die Hülsen 16 bzw. 17 sind vorzugsweise zylindrisch, sie können aber auch konisch ausgebildet sein. Die Verbindung ist eine feste Verbindung, das heisst die Hülsen 16 und 17 sind unverrückbar mit dem Körper 15 verbunden. Die Verbindung kann beispielsweise eine Klebeverbindung, eine Schweissverbindung oder eine Verbindung durch Formschluss sein. Möglich ist insbesondere ein Herstellung in einer Spritzgussmaschine. Die Hülsen 16 und 17 sind hierbei Einlegeteile im Werkzeug. Der Körper 15 wird dann an diese Einlegeteile angespritzt.

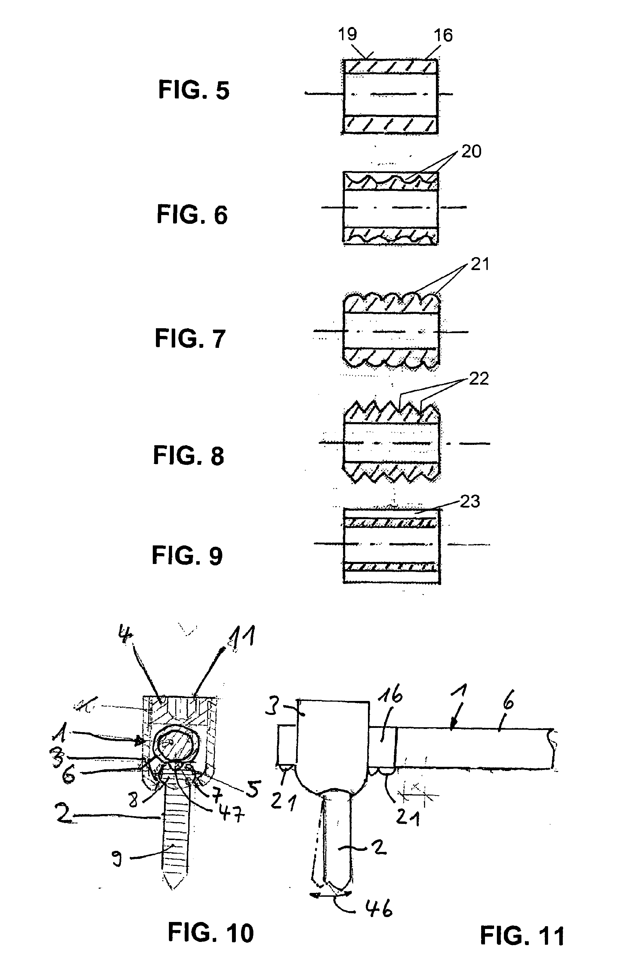

Die Hülsen 16 und 17 sind vorzugsweise aussenseitig strukturiert. Denkbar ist jedoch auch eine Hülse 16, die gemäss Figur 5 eine glatte Aussenseite 19 besitzt. Im Querschnitt kann diese Hülse 16 beispielsweise oval oder polygonal sein. Bei der Ausführung gemäss der Figur 6 ist die Aussenseite durch eine Mehrzahl von Vertiefungen 20 strukturiert. Diese Vertiefungen 20 können beispielsweise halbkugelförmig sein. Die Strukturierung kann aber auch gemäss Figur 7 durch Erhöhungen 21 realisiert werden, wobei diese beispielsweise ebenfalls halbkugelförmig sind. Bei der Ausführung gemäss der Figur 8 sind Vertiefungen 22 vorgesehen, welche umlaufende Rillen sind. Bei der Ausführung gemäss Figur 9 sind ebenfalls Rillen 23 vorgesehen, die sich jedoch axial über die gesamte Länge der Hülse 16 erstrecken. Die Hülse 17 kann entsprechend strukturiert sein. Die Hülsen 16 und 17 können gleich oder unterschiedliche strukturiert sein. Denkbar ist beispielsweise eine Ausführung, bei welcher eine der Hülsen 16 oder die Hülsen 16 und die Hülse 17 unterschiedliche strukturiert sind. Eine dieser Hülsen kann beispielsweise Rillen 22 gemäss Figur 8 und die andere Hülse Rillen 23 gemäss der Figur 9 aufweisen. Der Verbindungsstab 6 ist dann sowohl axial als auch radial fixier. Die Strukturierung erstreckt sich vorzugsweise über die gesamte Aussenseite der Hülse 16 bzw. 17. Denkbar ist jedoch auch eine Ausführung, bei welcher lediglich ein Teilbereich der Aussenseite strukturiert ist.

Die Figuren 10 und 11 zeigen die formschlüssige Verbindung eines Verbindungsstabes 6 mit der Klemmvorrichtung 3. Diese besitzt einen hülsenförmigen Träger 5 mit einer Öffnung 17, durch welche der Schaft 9 der Pedikelschraube 2 hindurchragt. Die Pedikelschraube 2 besitzt einen Kopf 8, der so im Träger 5 gelagert ist, dass die Pedikelschraube 2 im nicht geklemmten Zustand polygonal bewegbar ist, wie dies in Figur 11 mit Pfeil 46 angedeutet ist. Die polygonale Beweglichkeit ist jedoch nicht zwingend. Mittels eines Klemmelementes 4, beispielsweise einer Mutter mit einem Innensechskant 11, wird der Verbindungsstab 6 an den Kopf 8 angepresst. Im geklemmten Zustand ist die Pedikelschraube 2 bezüglich des Trägers 5 und auch bezüglich des Verbindungsstabes 6 unbeweglich. Der Schraubenkopf 8 besitzt an seinem Umfang wenigstens und vorzugsweise mehrere Vertiefungen 47, die korrespondierend zu den Erhöhungen 21 der Hülse 16 ausgebildet ist, an welcher der Verbindungsstab 6 geklemmt ist. Die Pedikelschraube 2 ist somit im geklemmtem Zustand formschlüssig mit dem Verbindungsstab 6 verbunden. Eine formschlüssige Verbindung ist auch zwischen dem Klemmelement 4 und dem Verbindungsstab 6 denkbar. Das Klemmelement 4 besitzt in diesem Fall wenigstens eine hier nicht gezeigte Vertiefung, in welche eine Erhöhung 21 der Hülse 16 eingreift. Dadurch ergibt sich eine noch festere Verbindung.

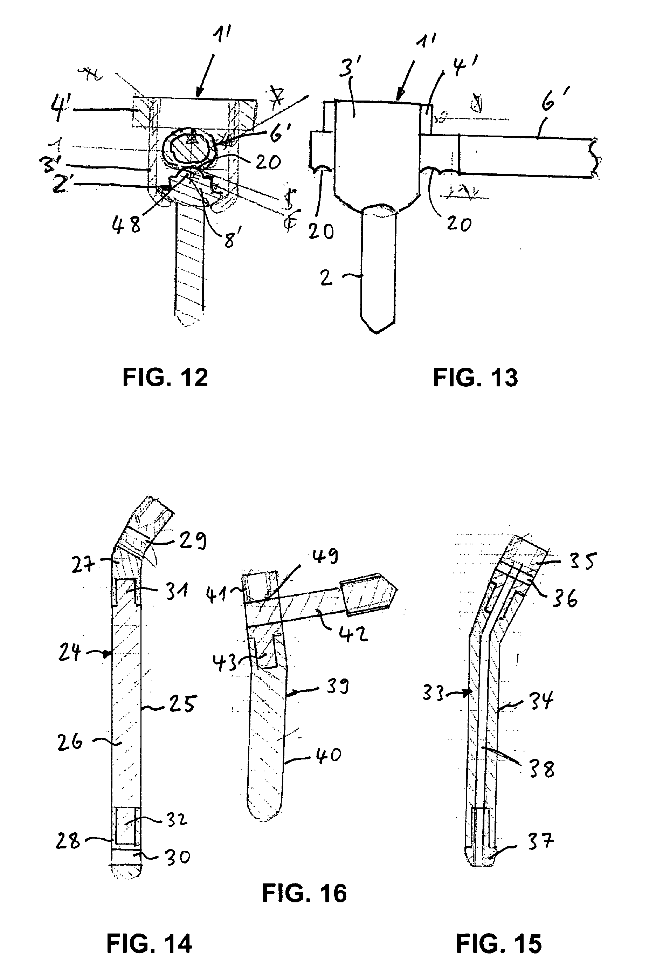

Bei der Ausführung gemäss den Figuren 12 und 13 besitzt ein Kopf 8' einer Pedikelschraube 2' eine Erhöhung 48, die in eine Vertiefung 20 eines Verbindungsstabes 6' eingreift. Auch hier ergibt sich entsprechend eine formschlüssige Verbindung zwischen der Pedikelschraube 2' und dem Verbindungsstab 6'. Die Klemmung des Verbindungsstabes 6' erfolgt hier mit einer Mutter 4' die aussenseitig auf die Klemmvorrichtung 3' aufgeschraubt ist. Die Figur 14 zeigt einen Marknagel 24, der ebenfalls ein Bauteil eines Implantatsystems ist und der einen länglichen Schaft 25 aufweist, der im wesentlichen durch einen Körper 26 aus Kunststoff gebildet wird. Der Körper 26 kann ebenfalls PEEK, PEK oder sonst ein geeigneter röntgentransparenter Kunststoff sein. Der Körper 26 ist über einen Zapfen 31 mit einem proximalen Element 27 fest verbunden, das ein Befestigungsloch 29 aufweist, an den der Marknagel 24 mit einem hier nicht gezeigten an sich üblichen Befestigungselement verbunden ist. Das proximale Element 27 ist ebenfalls aus einem Material hergestellt, das wesentlich härter ist als das Material des Körpers 26. Das Material kann ebenfalls Titan, eine Titanlegierung oder ein Implantatenstrahl sein. Der Körper 26 besitzt hier einen zweiten Zapfen 32, an dem ein distales Element 28 mit dem Körper 26 verbunden ist. Das distale Element 28 besitzt ein Befestigungsloch 30 für den Eingriff eines weiteren hier nicht gezeigten Befestigungselementes. Der Schaft 25 ist somit mit Ausnahme der Elemente 27 und 28 röntgentransparent. Die Befestigung belastet den Körper 26 nur unwesentlich. Trotzdem ist eine feste Verbindung und insbesondere Klemmverbindung zu den genannten Befestigungselementen möglich. Diese Verbindung kann beispielsweise eine Schraubverbindung oder Klemmverbindung sein.

Die Figur 15 zeigt ebenfalls einen Marknagel 33, der einen Schaft 34 aufweist, an den ein proximales Element 35 mit einem Befestigungsloch 36 und ein distales Element 37 angeordnet ist. Im Schaft 34 und in den Elementen 35 und 37 verläuft ein an sich bekannter Durchgang 38. Auch beim Marknagel 33 ist der Schaft 34 zwischen den Elementen 35 und 37 aus einem geeigneten Kunststoff hergestellt, der röntgentransparent ist.

Die Figur 16 zeigt einen Hüftnagel 39, der ebenfalls ein längliches Bauelement bildet, das mit einer Schenkelhalsschraube 42 verbunden ist. Ein Schaft 40 ist aus einem röntgentransparenten Kunststoff hergestellt und ist über einen Zapfen 43 mit einem distalen Element 41 verbunden. Dieses Element 41 besitzt ein Befestigungsloch 49, an dem der Hüftnagel 39 mit der Schenkelhalsschraube 42 verbunden ist. Das distale Element 41 besteht jedenfalls aus einem Material, das wesentlich härter ist als das Material des Schaftes 40. Die oben erwähnten Vorteile werden auch hier erreicht. Die Materialien können hier gleich sein wie sie bereits oben bezüglich der anderen Ausführungsbeispiele erwähnt ist worden sind. Denkbar sind jeweils an sich bekannte Marken, beispielsweise Bariumsulfat, Tantalfäden oder Kügelchen.

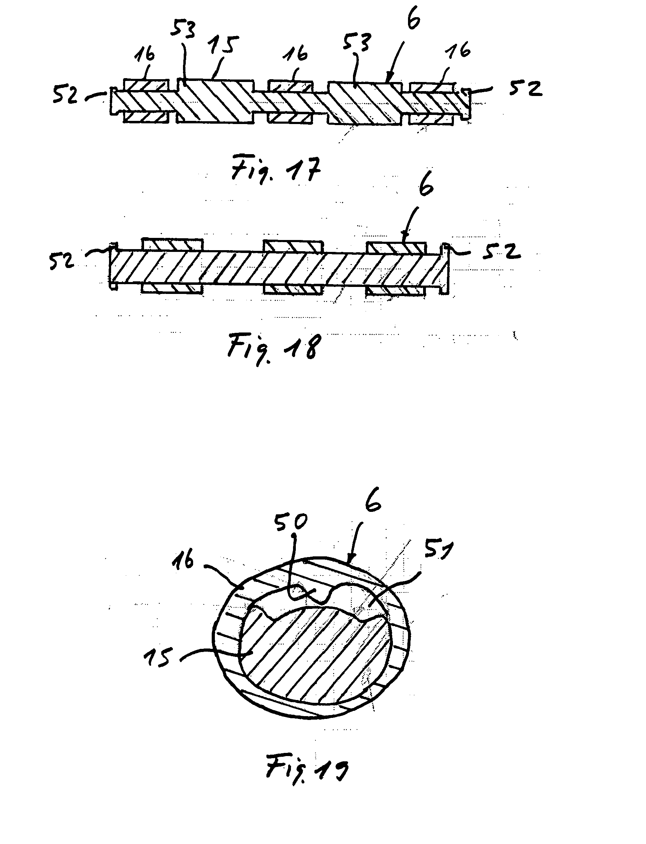

Die Figuren 17, 18 und 19 zeigen Verbindungsstäbe 6, die ein Implantatsystem, beispielsweise ein Pedikelsystem ermöglichen, das einen bedingt dynamischen Charakter besitzt. Mit einer solchen Dynamisierung kann die Kallusbildung gefördert und dadurch die Knochenbildung und somit eine allfällig gewünschte Fusion beschleunigt werden.

Bei der Ausführung nach Figur 17 ist ein Körper 15 vorgesehen, auf dem Hülsen 16 in Längsrichtung des Körpers 15 begrenzt verschieblich gelagert sind. Die Hülsen 16 können Umfangsrichtung des Körpers 15 auf diesem begrenzt oder unbegrenzt rotierbar gelagert sein. Die Figur 19 zeigt, wie eine Hülse 16 begrenzt rotierbar auf einem Körper 15 angeordnet sein kann. Die Hülse 16 besitzt an der Innenseite einen Noppen 50, der in eine Ausnehmung 51 des Körpers 15 eingreift. Der hier mögliche Drehwinkel ist wie ersichtlich durch Anschläge des Noppens 50 am Körper 15 begrenzt. Die Einschränkung der Rotationsfreiheit könnte auch durch andere Geometrien der Hülse 16 und des Körpers 15 erreicht werden. Beispielsweise könnten der Körper 15 und/oder die Hülse 16 mehrkantig ausgebildet sein.

Die Längsverschiebbarkeit der Hülsen 16 sind bei der Ausführung nach Figur 17 durch äussere Anschläge 52 und innere Anschläge 53 begrenzt. Bei der Ausführung nach Figur 18 sind die inneren Anschläge 53 weggelassen. Die äusseren Anschläge 52 sind jedoch nicht zwingend und könnten auch weggelassen werden.

Für die Hülsen 16 und den Körper 15 sind die oben erwähnten Materialien möglich. So können beispielsweise die Hülsen 16 aus Titan und der Körper 15 aus Kunststoff, beispielsweise PEEK hergestellt sein.

### Bezugszeichenhste

- 1: Stabilisierungsanordnung
- 2: Pedikelschraube
- 3: Klemmvorrichtung
- 4: Klemmelement
- 5: Träger
- 6: Verbindungsstab
- 7: Öffnung
- 8: Schraubenkopf
- 9: Schaft
- 10: Stabilisierungsanorndung
- 11: Innensechskant
- 12: Wirbelsäule
- 13: Wirbelkörper
- 14: Bandscheibe
- 15: Körper
- 16: Hülse
- 17: Hülse
- 18: Verdickung
- 19: Aussenseite
- 20: Vertiefung
- 21: Erhöhung
- 22: Vertiefung
- 23: Rille
- 24: Marknagel
- 25: Schaft
- 26: Körper
- 27: Proximales Element
- 28: DistalesElement
- 29: Befestigungsloch
- 30: Befestigungsloch
- 31: Zapfen
- 32: Zapfen
- 33: Marknagel
- 34: Schaft
- 35: Proximales Element
- 36: Befestigungsloch
- 37: Distales Element
- 38: Durchgang
- 39: Hüftnagel
- 40: Schaft
- 41: Distales Element
- 42: Schenkelhalsschraube
- 43: Zapfen
- 44: Aussenseite
- 45: Aussenseite
- 46: Pfeil
- 47: Vertiefung
- 48: Erhöhung
- 49: Befestigung
- 50: Noppen
- 51: Ausnehmung
- 52: Anschlag
- 53: Anschlag

## Patentansprüche

1. Implantatsystem zum Stabilisieren von Knochen, mit einem Bauteil (6, 24, 33, 39), das einen länglichen Körper (15, 25, 34, 40) aufweist, der wenigstens einen Abschnitt besitzt, an dem er mit einem anderen Teil (2, 3, 42), beispielsweise einer Pedikelschraube oder einer Klammer verbindbar ist, **dadurch gekennzeichnet, dass** der längliche Körper (15, 25, 34, 40) aus einem röntgentransparenten Kunststoff hergestellt und im genannten wenigstens einen Abschnitt fest mit einem weiteren Teil (16, 17, 27, 28, 35, 37, 41) verbunden ist, welcher eine Schnittstelle zum genannten anderen Teil (2, 42) bildet.

2. Implantatsystem nach Anspruch 1, **dadurch gekennzeichnet, dass** der weitere Teil (16, 27, 28, 35, 37, 41) an einem Ende des länglichen Körpers (15, 25, 34, 40) angeordnet ist

3. Implantatsystem nach Anspruch 1 oder 2, **dadurch gekennzeichnet, dass** der röntgentransparente Kunststoff ein E-Modul aufweist, das sich vom E-Modul des weiteren Teils (16, 17, 27, 28, 35, 37, 41) unterscheidet.

4. Implantatsystem nach einem der Ansprüche 1 bis 3, **dadurch gekennzeichnet, dass** der weitere Teils (16, 17, 27, 28, 35, 37, 41) aus Metall, insbesondere Titan, einer Titanlegierung, einem Implantatenstahl oder PEEK hergestellt ist.

5. Implantatsystem nach einem der Ansprüche 1 bis 4, **dadurch gekennzeichnet, dass** der weitere Teil (16, 17, 27, 28, 37) hülsenförmig ausgebildet ist.

6. Implantatsystem nach einem der Ansprüche 1 bis 6, **dadurch gekennzeichnet, dass** der weitere Teil (16, 17) aussenseitig für einen Formschluss mit einem Befestigungselement (2, 3) strukturiert ist.

7. Implantatsystem nach Anspruch 6, **dadurch gekennzeichnet, dass** die Strukturierung eine Mehrzahl von Vertiefungen (20, 22, 23) oder Erhöhungen (21) umfasst.

8. Implantatsystem nach einem der Ansprüche 1 bis 7, **dadurch gekennzeichnet, dass** an beiden Enden des länglichen Körpers (15, 25, 34, 40) jeweils ein weiterer Teil (16, 27, 28, 35, 37) angeordnet ist.

9. Implantatsystem nach einem der Ansprüche 1 bis 8, **dadurch gekennzeichnet, dass** das Bauteil ein Verbindungsstab (6) zum Verbinden von Wirbelkörpern (13), ein Marknagel (24, 33, 39) oder ein Verbindungselement eines externen Fixateurs ist.

10. Implantatsystem nach einem der Ansprüche 1 bis 9, **dadurch gekennzeichnet, dass** der längliche Körper (15, 25, 34, 40) aus einem faserverstärkten Kunststoff, beispielsweise faserverstärktem PEEK hergestellt ist.

11. Implantatsystem nach einem der Ansprüche 1 bis 10, **dadurch gekennzeichnet, dass** das Bauteil ein Verbindungsstab (6) ist und dass das Befestigungselement ein Anker (2, 3) ist, der mit einem Wirbelkörper (13) verbindbar ist und der Klemmmittel (4) aufweist, die an den härteren Teil (16, 17) anpressbar sind.

12. Implantatsystem nach Anspruch 11, **dadurch gekennzeichnet, dass** der weitere Teil (16, 17) aussenseitig für einen Formschluss strukturiert ist und dass die wenigstens eine Klemmvorrichtung (3) ein Klemmelement (4) aufweist, das einen zur Strukturierung korrespondierenden Bereich für den Formschluss aufweist.

13. Implantatsystem nach Anspruch 9, **dadurch gekennzeichnet, dass** der Marknagel (24, 33, 39) wenigstens an einem distalen und/oder proximalen Ende einen härteren Teil (27, 28, 35, 41) aufweist, in den ein Befestigungsloch (29, 30, 36) oder ein anderes Befestigungsmittel eingearbeitet ist.

14. Implantatsystem nach einem der Ansprüche 1 bis 14, **dadurch gekennzeichnet, dass** der weitere Teil bezüglich des länglichen Körpers beweglich ist.

15. Implantatsystem nach Anspruch 14, **dadurch gekennzeichnet, dass** der weitere Teil (16) axial und/oder radial begrenzt beweglich auf dem länglichen Körper (15) gelagert ist.
